# EUROPEAN PATENT APPLICATION

(11) **EP 4 516 912 A1**
(43) Date of publication of application: **05.03.2025**
(21) Application number: 23194970.2
(22) Date of filing: 01.09.2023
(51) Int. Cl.: C12N 15/82

(54) **HERBICIDE RESISTANT PLANTS**

(71) Applicant: Eberhard-Karls-Universität Tübingen, 72074 Tübingen (DE)
(72) Inventor: HARTER, Klaus, 72074 Tübingen (DE); SAILE, Svenja, 72074 Tübingen (DE)
(74) Representative: Kuttenkeuler, David

(57) **Abstract**

The invention is based on a surprising finding that overexpression of an enzyme of the shikimate pathway confers herbicide resistance to 7-deoxy-sedoheptulose (7dSh), while not directly binding or catalytically targeting the molecule. The invention is directed at methods for the selection or production of herbicide resistant or tolerant plants and methods for controlling unwanted vegetation on a crop field. Further, the invention provides combinations of the plant products produced or obtained with the invention and a herbicide composition, as well as uses thereof.

## Description

### FIELD OF THE INVENTION

The invention is based on a surprising finding that overexpression of an enzyme of the shikimate pathway confers herbicide resistance to 7-deoxy-sedoheptulose (7dSh), while not directly binding or catalytically targeting the molecule. The invention is directed at methods for the selection or production of herbicide resistant or tolerant plants and methods for controlling unwanted vegetation on a crop field. Further, the invention provides combinations of the plant products produced or obtained with the invention and a herbicide composition, as well as uses thereof.

### DESCRIPTION

The Shikimate pathway has been a major target for weed controlling agents, with glyphosate as one of its widely used herbicide agents. Glyphosate blocks 5- enolpyruvylshikimat-3-phosphat-synthase (EPSPS, which is the sixth enzyme of the shikimate pathway). While risk assessment studies point in a different direction, there is an increasing public disquiet that glyphosate might be cancerogenic and might harm plants and animals. Therefore, a need exists to provide novel herbicides, which are not cancerogenic or detrimental in any other way to human beings and are not harmful for useful plants and animals.

The shikimate pathway is a crucial metabolic route in plants responsible for the biosynthesis of aromatic amino acids and other important metabolites. It starts with the conversion of phosphoenolpyruvate (PEP) and erythrose-4-phosphate (E4P) to form 3-deoxy-D-arabino-heptulosonate-7-phosphate (DAHP). This initial step is considered the rate-limiting step of the pathway and is catalyzed by the enzyme DAHP synthase. Subsequent enzymatic reactions convert DAHP into chorismate as final product of the pathway. Chorismate a key intermediate in the synthesis of aromatic amino acids such as phenylalanine, tyrosine, and tryptophan. Chorismate is also a precursor for the production of other essential metabolites, such as certain vitamins, cofactors, and secondary metabolites in plants. The shikimate pathway plays a vital role in plant growth, development, and defense mechanisms, as aromatic amino acids are essential for protein synthesis and various other physiological processes. Additionally, some compounds derived from the shikimate pathway are involved in the synthesis of secondary metabolites with diverse functions, including defense against pathogens and attraction of pollinators. Because of its importance in plant metabolism, the shikimate pathway is a target for the development of herbicides and antimicrobial agents.

Brilisauer et al 2019 (Nat Commun. 2019; 10: 5) found that the bacterial metabolite 7-deoxy-sedoheptulose (7dSh) effectively blocks the shikimate pathway, disrupting the production of vital plant metabolites. By inhibiting this pathway, the compound hinders the growth and survival of prototrophic organisms that rely on it for essential nutrient synthesis. 7dSh shows potential as a natural and eco-friendly alternative for controlling the growth of certain organisms that may be harmful in various applications, such as agriculture, biotechnology, and environmental management. While Brilisauer et al found the 7dSh treatment in plants results in an accumulation of 3-deoxy-d-*arabino*-heptulosonate 7-phosphate (DAHP), the specific target of 7dSh remains unknown.

Hence, the mode of action of 7dSh is still poorly understood, and its application as a herbicide in crop protection still requires the development of strategies to increase crop plant tolerance to 7dSh. The investigation of the herbicidal effect of 7dSh on the model and crop plants and weeds, namely thale cress (Arabidopsis thaliana), rapeseed/canola (Brassica napus), barley (Hordeum vulgare), maize (Zea mays), red-root amaranth (Amaranthus retroflexus) and velvetleaf (Abutilon theophrasti) by the current inventors demonstrate that individuals of these plant species reacted at an application concentration of 100 to 500 µM 7dSh in the growth medium with a strong inhibition of root growth, which was reflected in a significant reduction of further development of the plants. The effective concentrations used are comparable to those of glyphosate, which was tested in parallel.

According to current data of the inventors, the plants take up 7dSh into the roots by means of sugar transport. However, in contrast to glyphosate, the 7dSh taken-up is, though transported into the aboveground plant parts, not active in the aboveground plant parts (shoot, leaves), but possesses its activity in the underground organ only. These results explain the strong herbicidal effect of 7dSh directly on root growth, which indirectly has a negative effect on the further development of the plant through reduced water and nutrient supply. Consequently, the present invention focuses on the development of a granular formulation of 7dSh that enables the use of the active ingredient as a "pre-emergence" herbicide.

Thus, it is an object of the invention to develop crop plants having an increased resistance to the antimetabolite effects of 7dSh, and thereby to provide new strategies to use 7dSh as a commercial herbicide.

### BRIEF DESCRIPTION OF THE INVENTION

Generally, and by way of brief description, the main aspects of the present invention can be described as follows:
In **a first aspect,** the invention pertains to a method for controlling unwanted vegetation on a crop field used for growing a crop plant, the method comprising the steps of:
   - a crop plant from a crop plant species or from a crop plant species variety of a desired crop having a detectable expression of a 3-dehydroquinate synthase protein;
   - Growing the selected crop plant on the field;
   - Controlling unwanted vegetation on the field by using 7dSh, or a derivative thereof, on the field.
In **a second aspect,** the invention pertains to a method for producing a herbicide resistant crop plant, the method comprising the steps of
   - Introducing transgenic constructs for the expression of a 3-dehydroquinate synthase protein (DHQS) in a crop plant into multiple candidate crop plants or crop plant species;
   - Detecting the expression of the 3-dehydroquinate synthase protein in the candidate crop plants;
   - Selecting the crop plant with the highest expression of the 3-dehydroquinate synthase protein and/or with highest and most root-restricted expression of the 3-dehydroquinate synthase protein.
In **a third aspect,** the invention pertains to a plant cell nucleus, a plant cell, a plant tissue, propagation material, seed, pollen, progeny, or a plant part, resulting in a plant with increased herbicide tolerance or resistance after regeneration; or a plant with increased herbicide tolerance or resistance; or a part thereof; with said herbicide tolerance or resistance increased as compared to a corresponding wild type produced by a method as defined herein or being transformed with the nucleic acid molecule encoding for a 3-dehydroquinate synthase protein.
In **a fourth aspect,** the invention pertains to a transgenic plant comprising one or more of plant cell nuclei or plant cells, progeny, seed or pollen or produced by a transgenic plant of the invention.
In **a fifth aspect,** the invention pertains to a method for growing the plant as defined in any one of claims 15 to 20, while controlling weeds in the vicinity of said plant, said method comprising the steps of:
   - growing said plant on a field; and
   - applying a herbicide composition comprising a 7dSh, or a derivative thereof, on the field, wherein the herbicide normally inhibits the nutrient uptake through the roots of a plant, at a level of the herbicide that would inhibit the growth of a corresponding wild-type plant.
In **a sixth aspect,** the invention pertains to a combination useful for weed control, comprising (a) a nucleic acid molecule for a transgenic expression of the 3-dehydroquinate synthase protein, which nucleic acid molecule is capable of being expressed in the roots of a crop plant to thereby provide to that crop plant a tolerance to a 7dSh, or a derivative thereof, containing herbicide composition; and (b) a herbicide composition comprising 7dSh, or a derivative thereof.
In **a seventh aspect,** the invention pertains to a use of a combination useful for weed control, comprising (a) a nucleic acid molecule for a transgenic expression of the 3-dehydroquinate synthase protein, which nucleic acid molecule is capable of being expressed in the roots of a crop plant to thereby provide to that crop plant tolerance to a 7dSh, or a derivative thereof, containing herbicide composition; and (b) a herbicide composition comprising 7dSh, or a derivative thereof, to control weeds at a plant cultivation site.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The present invention is predicated upon the surprising finding that while a certain homolog of plant enzyme 3-dehydroquinate synthase (DHQS) is not directly inhibited in its enzymatic activity by the herbicide 7dSh, overexpression of said homolog still confers resistance to the antimetabolite effects of 7dSh. Therefore, the present invention is defined by the following aspects and embodiments, which are to be understood in context of the complete description, and the appended examples, figures and claims.

A "transgenic plant", as used herein, refers to a plant which contains a foreign nucleotide sequence inserted into either its nuclear genome or organelle genome. It encompasses further the offspring generations i.e. the T1-, T2- and consecutively generations or BC1-, BC2- and consecutively generation as well as crossbreeds thereof with non-transgenic or other transgenic plants. A transgenic plant may comprise nucleotide sequences which are introduced but which are identical in sequence to endogenous sequences, for example for a controlled overexpression of endogenous genes.

The term "organelle" according to the invention shall mean for example "mitochondria", "plastid" or endoplasmic reticulum (ER). The term "plastid" according to the invention is intended to include various forms of plastids including proplastids, chloroplasts, chromoplasts, gerontoplasts, leucoplasts, amyloplasts, elaioplasts and etioplasts, preferably chloroplasts. They all have as a common ancestor the aforementioned proplasts.

The term "introduced" in the context of this specification shall mean the insertion of a nucleic acid sequence into the organism by means of a "transfection", "transduction" or preferably by "transformation".

A plastid, such as a chloroplast, has been "transformed" by an exogenous (preferably foreign) nucleic acid sequence if nucleic acid sequence has been introduced into the plastid that means that this sequence has crossed the membrane or the membranes of the plastid. The foreign DNA may be integrated (covalently linked) into plastid DNA making up the genome of the plastid, or it may remain not integrated (e.g., by including a chloroplast origin of replication). "Stably" integrated DNA sequences are those, which are inherited through plastid replication, thereby transferring new plastids, with the features of the integrated DNA sequence to the progeny.

As used herein, "plant" is meant to include not only a whole plant but also a part thereof i.e., one or more cells, and tissues, including for example, leaves, stems, shoots, roots, flowers, fruits and seeds.

The term "herbicide tolerance or resistance" as used herein it is intended that a plant that is tolerant or resistant to at least one herbicide at a level that would normally kill, or inhibit the growth of, a normal or wild-type plant. That herbicide is preferably 7dSh, or a derivative thereof.

Any increase in herbicide tolerance or resistance is an improved herbicide tolerance or resistance in accordance with the invention. For example, the improvement in herbicide tolerance or resistance can comprise a 1.5×, 2×, 2.5×, 3×, 5×, 10×, 20×, 30×, 40×, 50×, 75×, 100×, 150×, 200× or greater increase in any measurable parameter. Such parameter is preferably a plant growth parameter.

Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms "gene(s)", "polynucleotide", "nucleic acid sequence", "nucleotide sequence", or "nucleic acid molecule(s)" as used herein include known types of modifications, for example, methylation, "caps", substitutions of one or more of the naturally occurring nucleotides with an analogue. Preferably, the DNA or RNA sequence comprises a coding sequence encoding the herein defined polypeptide.

As also used herein, the terms "nucleic acid" and "nucleic acid molecule" are intended to include DNA molecules (e.g. cDNA or genomic DNA) and RNA molecules (e.g. mRNA) and analogs of the DNA or RNA generated using nucleotide analogs. The nucleic acid molecule can be single-stranded or double-stranded.

An "isolated" nucleic acid molecule is one that is substantially separated from other nucleic acid molecules, which are present in the natural source of the nucleic acid. That means other nucleic acid molecules are present in an amount less than 5% based on weight of the amount of the desired nucleic acid, preferably less than 2% by weight, more preferably less than 1% by weight, most preferably less than 0.5% by weight. Preferably, an "isolated" nucleic acid is free of some of the sequences that naturally flank the nucleic acid (i.e., sequences located at the 5' and 3' ends of the nucleic acid) in the genomic DNA of the organism from which the nucleic acid is derived. For example, in various embodiments, the isolated herbicide resistance and/or tolerance related protein encoding nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

A "coding sequence" is a nucleotide sequence, which is transcribed into an RNA, e.g. a regulatory RNA, such as a miRNA, a ta-sRNA, co-suppression molecule, an RNAi, a ribozyme, etc. or into a mRNA which is translated into a polypeptide when placed under the control of appropriate regulatory sequences. The boundaries of the coding sequence are determined by a translation start codon at the 5' -terminus and a translation stop codon at the 3' -terminus. A coding sequence can include, but is not limited to mRNA, cDNA, recombinant nucleotide sequences or genomic DNA, while introns may be present as well under certain circumstances.

As used in the present context a nucleic acid molecule may also encompass the untranslated sequence located at the 3' and at the 5' end of the coding gene region, for example 2000, preferably less, e.g. 500, preferably 200, especially preferable 100, nucleotides of the sequence upstream of the 5' end of the coding region and for example 300, preferably less, e.g. 100, preferably 50, especially preferable 20, nucleotides of the sequence downstream of the 3' end of the coding gene region.

"Polypeptide" refers to a polymer of amino acid (amino acid sequence) and does not refer to a specific length of the molecule. Thus, peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring. An "isolated" polynucleotide or nucleic acid molecule is separated from other polynucleotides or nucleic acid molecules, which are present in the natural source of the nucleic acid molecule. An isolated nucleic acid molecule may be a chromosomal fragment of several kb, or preferably, a molecule only comprising the coding region of the gene. Accordingly, an isolated nucleic acid molecule of the invention may comprise chromosomal regions, which are adjacent 5 ' and 3' or further adjacent chromosomal regions, but preferably comprises no such sequences which naturally flank the nucleic acid molecule sequence in the genomic or chromosomal context in the organism from which the nucleic acid molecule originates (for example sequences which are adjacent to the regions encoding the 5' - and 3' -UTRs of the nucleic acid molecule). An "isolated" or "purified" polypeptide or biologically active portion thereof is free of some of the cellular material when produced by recombinant DNA techniques, or chemical precursors or other chemicals when chemically synthesized. The language "substantially free of cellular material" includes preparations of a protein in which the polypeptide is separated from some of the cellular components of the cells in which it is naturally or recombinantly produced.

"Amount of protein or mRNA" is understood as meaning the molecule number of polypeptides or mRNA molecules in an organism, especially a plant, a tissue, a cell or a cell compartment. "Increase" in the amount of a protein means the quantitative increase of the molecule number of said protein in an organism, especially a plant, a tissue, a cell or a cell compartment such as an organelle like a plastid or mitochondria or part thereof-for example by one of the methods described herein below-in comparison to a wild type, control or reference.

The increase in molecule number amounts preferably to 1% or more, preferably to 10% or more, more preferably to 30% or more, especially preferably to 50%, 70% or more, very especially preferably to 100%, most preferably to 500% or more. However, a de novo expression is also regarded as subject of the present invention. Such increase amount of protein is preferably of a DHQS protein which is a homolog of the protein of SEQ ID NO: 1 to 4, preferably SEQ ID NO 1.

The terms "wild type", "control" or "reference" are exchangeable and can be a cell or a part of organisms such as an organelle like a chloroplast or a tissue, or an organism, in particular a plant, which was not modified or treated according to the herein described process according to the invention. Accordingly, the cell or a part of organisms such as an organelle like a chloroplast or a tissue, or an organism, in particular a plant used as wild type, control or reference corresponds to the cell, organism, plant or part thereof as much as possible and is in any other property but in the result of the process of the invention as identical to the subject matter of the invention as possible. Thus, the wild type, control or reference is treated identically or as identical as possible, saying that only conditions or properties might be different which do not influence the quality of the tested property.

Preferably, any comparison is carried out under analogous conditions. The term "analogous conditions" means that all conditions such as, for example, culture or growing conditions, soil, nutrient, water content of the soil, temperature, humidity or surrounding air or soil, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, an organism, in particular a plant, which was not modified or treated according to the herein described process of the invention and is in any other property as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or - organism, in particular plant, relates to an organelle, cell, tissue or organism, in particular plant, which is nearly genetically identical to the organelle, cell, tissue or organism, in particular plant, of the present invention or a part thereof preferably 90% or more, e.g. 95%, more preferred are 98%, even more preferred are 99,00%, in particular 99,10%, 99,30%, 99,50%, 99,70%, 99,90%, 99,99%, 99,999% or more. Most preferable the "reference", "control", or "wild type" is a subject, e.g. an organelle, a cell, a tissue, an organism, in particular a plant, which is genetically identical to the organism, in particular plant, cell, a tissue or organelle used according to the process of the invention except that the responsible or activity conferring nucleic acid molecules or the gene product encoded by them are amended, manipulated, exchanged or introduced according to the inventive process. In case, a control, reference or wild type differing from the subject of the present invention only by not being subject of the process of the invention can not be provided, a control, reference or wild type can be an organism in which the cause for the modulation of an activity conferring the enhanced tolerance or resistance to herbicides as compared to a corresponding, e.g. non-transformed, wild type plant cell, plant or part thereof or expression of the nucleic acid molecule of the invention as described herein has been switched back or off, e.g. by knocking out the expression of responsible gene product, e.g. by antisense or RNAi or miRNA inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can for example be knocked out by introducing inactivating point mutations, which lead to an enzymatic activity inhibition or a destabilization or an inhibition of the ability to bind to cofactors etc. Accordingly, preferred reference subject is the starting subject of the present process of the invention. Preferably, the reference and the subject matter of the invention are compared after standardization and normalization, e.g. to the amount of total RNA, DNA, or protein or activity or expression of reference genes, like housekeeping genes, such as ubiquitin, actin or ribosomal proteins.

The term "expression" refers to the transcription and/or translation of a codogenic gene segment or gene. As a rule, the resulting product is an mRNA or a protein.

The increase or modulation according to this invention can be constitutive, e.g. due to a stable permanent transgenic expression or to a stable mutation in the corresponding endogenous gene encoding the nucleic acid molecule of the invention or to a modulation of the expression or of the behavior of a gene conferring the expression of the polypeptide of the invention, or transient, e.g. due to an transient transformation or temporary addition of a modulator such as a agonist or antagonist or inducible, e.g. after transformation with a inducible construct carrying the nucleic acid molecule of the invention under control of a inducible promoter and adding the inducer, e.g. tetracycline or as described herein below.

Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by 10% or more, advantageously 20%, 30% or 40% or more, especially advantageously by 50%, 60% or 70% or more in comparison with the starting organism. This leads to increased herbicide tolerance or resistance, as compared to a corresponding, e.g. non-transformed, wild type plant or part thereof.

The increase in activity of the polypeptide amounts in a cell, a tissue, an organelle, an organ or an organism, preferably a plant, or a part thereof preferably to 5% or more, preferably to 20% or to 50%, especially preferably to 70%, 80%, 90% or more, very especially preferably are to 100%, 150% or 200%, most preferably are to 250% or more in comparison to the control, reference or wild type. In one embodiment the term increase means the increase in amount in relation to the weight of the organism or part thereof (w/w).

By "vectors" is meant with the exception of plasmids all other vectors known to those skilled in the art such as by way of example phages, viruses such as SV40, CMV, baculovirus, adenovirus, transposons, IS elements, phasmids, phagemids, cosmids, linear or circular DNA. These vectors can be replicated autonomously in the host organism or be chromosomally replicated, chromosomal replication being preferred. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g. bacterial vectors having a bacterial origin of replication and episomal mammalian vectors). Other vectors (e.g. non-episomal mammalian vectors) are integrated into the genome of a host cell or a organelle upon introduction into the host cell, and thereby are replicated along with the host or organelle genome. Moreover, certain vectors are capable of directing the expression of genes to which they are operatively linked. Such vectors are referred to herein as "expression vectors." In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses, and adeno-associated viruses), which serve equivalent functions.

As used herein, "operatively linked" is intended to mean that the nucleotide sequence of interest is linked to the regulatory sequence(s) in a manner which allows for expression of the nucleotide sequence (e.g. in an in vitro transcription/translation system or in a host cell when the vector is introduced into the host cell). The term "regulatory sequence" is intended to include promoters, enhancers, and other expression control elements (e.g. polyadenylation signals). Such regulatory sequences are described, for example, in Goeddel, Gene Expression Technology: Methods in Enzymology 185, Academic Press, San Diego, Calif. (1990), and Gruber and Crosby, in: Methods in Plant Molecular Biology and Biotechnology, eds. Glick and Thompson, Chapter 7, 89-108, CRC Press; Boca Raton, Fla., including the references therein. Regulatory sequences include those that direct constitutive expression of a nucleotide sequence in many types of host cells and those that direct expression of the nucleotide sequence only in certain host cells or under certain conditions.

"Transformation" is defined herein as a process for introducing heterologous DNA into a plant cell, plant tissue, or plant. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but is not limited to, viral infection, electroporation, lipofection, and particle bombardment. Such "transformed" cells include stably transformed cells in which the inserted DNA is capable of replication either as an autonomously replicating plasmid or as part of the host chromosome. They also include cells which transiently express the inserted DNA or RNA for limited periods of time. Transformed plant cells, plant tissue, or plants are understood to encompass not only the end product of a transformation process, but also transgenic progeny thereof.

The terms "transformed," "transgenic," and "recombinant" refer to a host organism such as a bacterium or a plant into which a heterologous nucleic acid molecule has been introduced. The nucleic acid molecule can be stably integrated into the genome of the host or the nucleic acid molecule can also be present as an extra-chromosomal molecule. Such an extra-chromosomal molecule can be auto-replicating. Transformed cells, tissues, or plants are understood to encompass not only the end product of a transformation process, but also transgenic progeny thereof. A "non-transformed", "non-transgenic" or "non-recombinant" host refers to a wild-type organism, e.g. a bacterium or plant, which does not contain the heterologous nucleic acid molecule.

The terms "host organism", "host cell", "recombinant (host) organism" and "transgenic (host) cell" are used here interchangeably. Of course these terms relate not only to the particular host organism or the particular target cell but also to the descendants or potential descendants of these organisms or cells. Since, due to mutation or environmental effects certain modifications may arise in successive generations, these descendants need not necessarily be identical with the parental cell but nevertheless are still encompassed by the term as used here.

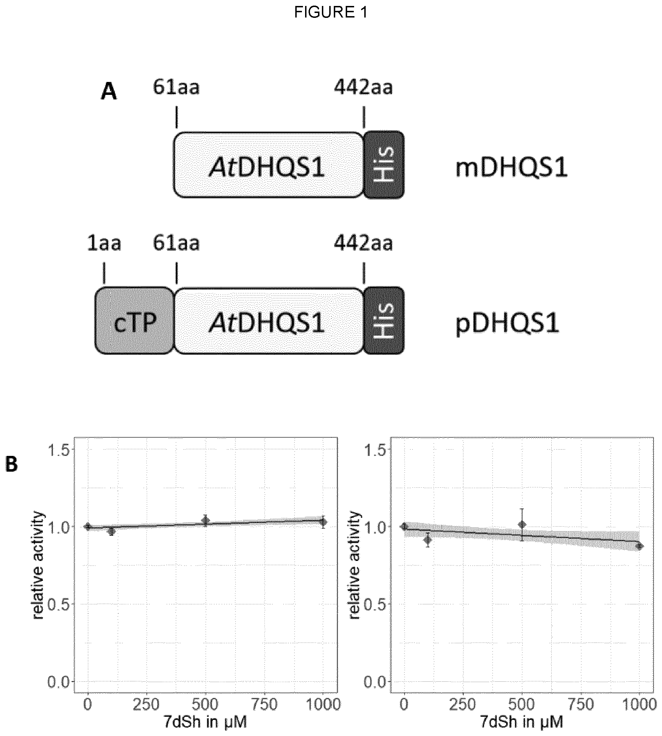

A 7dSh in accordance with the invention is a compound as disclosed in Rath P et al ChemBioChem 2022, e202200241 (incorporated herein in its entirety). A derivative of 7dSh is preferably selected from a derivative wherein one or two hydroxygroups are modified, for example are modified in their steric conformation (is a sterically isomeric compound to 7dSh), or is substituted for example with a halogen such as F. Most preferably a derivative of 7dSh is a compound selected from 7d7FSh, 5,7dd5Flh, 7d5MSh, 7dGh, and 5,7ddGh. Such compounds are disclosed in Figure 1A of Rath P et al ChemBioChem 2022, e202200241, which is incorporated herein.

For the purposes of the invention "transgenic"or "recombinant"means with regard for example to a nucleic acid sequence, an expression cassette (=gene construct, nucleic acid construct) or a vector containing the nucleic acid sequence according to the invention or an organism transformed by said nucleic acid sequences, expression cassette or vector according to the invention all those constructions produced by genetic engineering methods in which either
(a) the nucleic acid sequence comprising the sequence encoding any one of SEQ ID NO: 1 to 4, preferably SEQ ID NO 1, or a homolog thereof, or its derivatives or parts thereof; or
(b) a genetic control sequence functionally linked to the nucleic acid sequence described under (a), for example a 3' - and/or 5' -genetic control sequence such as a promoter or terminator, or
(c) (a) and (b);
are not found in their natural, genetic environment or have been modified by genetic engineering methods, wherein the modification may by way of example be a substitution, addition, deletion, inversion or insertion of one or more nucleotide residues.

"Natural genetic environment" means the natural genomic or chromosomal locus in the organism of origin or inside the host organism or presence in a genomic library. In the case of a genomic library the natural genetic environment of the nucleic acid sequence is preferably retained at least in part. The environment borders the nucleic acid sequence at least on one side and has a sequence length of at least 50 bp, preferably at least 500 bp, particularly preferably at least 1,000 bp, most particularly preferably at least 5,000 bp. A naturally occurring expression cassette-for example the naturally occurring combination of the natural promoter of the nucleic acid sequence according to the invention with the corresponding gene-turns into a transgenic expression cassette when the latter is modified by unnatural, synthetic ("artificial") methods such as by way of example a mutagenation.

The term "transgenic plants" used in accordance with the invention also refers to the progeny of a transgenic plant, for example the T1, T2, T3 and subsequent plant generations or the BC1, BC2, BC3 and subsequent plant generations. Thus, the transgenic plants according to the invention can be raised and selfed or crossed with other individuals in order to obtain further transgenic plants according to the invention. Transgenic plants may also be obtained by propagating transgenic plant cells vegetatively. The present invention also relates to transgenic plant material, which can be derived from a transgenic plant population according to the invention. Such material includes plant cells and certain tissues, organs and parts of plants in all their manifestations, such as seeds, leaves, anthers, fibers, tubers, roots, root hairs, stems, embryo, calli, cotelydons, petioles, harvested material, plant tissue, reproductive tissue and cell cultures, which are derived from the actual transgenic plant and/or can be used for bringing about the transgenic plant. Any transformed plant obtained according to the invention can be used in a conventional breeding scheme or in in vitro plant propagation to produce more transformed plants with the same characteristics and/or can be used to introduce the same characteristic in other varieties of the same or related species. Such plants are also part of the invention. Seeds obtained from the transformed plants genetically also contain the same characteristic and are part of the invention. As mentioned before, the present invention is in principle applicable to any plant and crop that can be transformed with any of the transformation method known to those skilled in the art.

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalent have the similar immunological characteristic, e.g. comprise similar epitopes.

As used herein, the terms "gene" and "recombinant gene" refer to nucleic acid molecules comprising an open reading frame encoding the polypeptide of the invention or comprising the nucleic acid molecule of the invention or encoding the polypeptide used in the process of the present invention, preferably from a crop plant or from a microorganism useful for the method of the invention. Such natural variations can typically result in 1 to 5% variance in the nucleotide sequence of the gene. Any and all such nucleotide variations and resulting amino acid polymorphisms in genes encoding a polypeptide of the invention or comprising the nucleic acid molecule of the invention that are the result of natural variation and that do not alter the functional activity as described are intended to be within the scope of the invention.

The term "control of undesired vegetation" is to be understood as meaning the controlling, particularly killing of weeds and/or otherwise retarding or inhibiting the normal growth of the weeds. Weeds, in the broadest sense, are understood as meaning all those plants which grow in locations where they are undesired, e.g. at a plant cultivation site. The weeds of the present invention include, for example, dicotyledonous and monocotyledonous weeds. Dicotyledonous weeds include, but are not limited to, weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, and Taraxacum. Monocotyledonous weeds include, but are not limited to, weeds of of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, and Apera. In addition, the weeds of the present invention can include, for example, crop plants that are growing in an undesired location. For example, a volunteer maize plant that is in a field that predominantly comprises soybean plants can be considered a weed, if the maize plant is undesired in the field of soybean plants.

Generally, the term "herbicide" is used herein to mean an active ingredient that kills, controls or otherwise adversely modifies the growth of plants. The preferred amount or concentration of the herbicide is an "effective amount" or "effective concentration." By "effective amount" and "effective concentration" is intended an amount and concentration, respectively, that is sufficient to kill or inhibit the growth of a similar, wild-type, plant, plant tissue, plant cell, microspore, or host cell, but that said amount does not kill or inhibit as severely the growth of the herbicide-resistant plants, plant tissues, plant cells, microspores, and host cells of the present invention. Typically, the effective amount of a herbicide is an amount that is routinely used in agricultural production systems to kill weeds of interest. Such an amount is known to those of ordinary skill in the art, or can be easily determined using methods known in the art. Furthermore, it is recognized that the effective amount of an herbicide in an agricultural production system might be substantially different than an effective amount of an herbicide for a plant culture system such as, for example, the microspore culture system. Herbicidal activity is exhibited by herbicides useful for the the present invention when they are applied directly to the plant or to the locus of the plant at any stage of growth or before planting or emergence. The effect observed depends upon the plant species to be controlled, the stage of growth of the plant, the application parameters of dilution and spray drop size, the particle size of solid components, the environmental conditions at the time of use, the specific compound employed, the specific adjuvants and carriers employed, the soil type, and the like, as well as the amount of chemical applied. These and other factors can be adjusted as is known in the art to promote non-selective or selective herbicidal action. Generally, it is preferred to apply the herbicide postemergence to relatively immature undesirable vegetation to achieve the maximum control of weeds. A preferred herbicide is a 7dHs, or a derivative thereof.

### Specific aspects and embodiments of the invention

In **a first aspect,** the invention pertains to a method for controlling unwanted vegetation on a crop field used for growing a crop plant, the method comprising the steps of:
- a crop plant from a crop plant species or from a crop plant species variety of a desired crop having a detectable expression of a 3-dehydroquinate synthase protein;
- Growing the selected crop plant on the field;
- Controlling unwanted vegetation on the field by using 7dSh, or a derivative thereof, on the field.

Preferably, the 3-dehydroquinate synthase protein is a homolog, paralog or ortholog of Arabidosis thaliana 3-dehydroquinate synthase 1 (AtDHQS1), or is a protein consisting of an amino acid sequence with at least 80%, 85%, 90%, 95%, 96%, 97%, 98%, or 99% sequence identity to an AtDHQS1 (SEQ ID NO: 1).

Preferably, the 3-dehydroquinate synthase protein is a homolog, paralog or ortholog of DHQS1 of any plant species, or is a protein consisting of an amino acid sequence with at least 80%, 85%, 90%, 95%, 96%, 97%, 98%, or 99% sequence identity to an DHQS1 shown in any one of SEQ ID NO: 1 to 4.

In some preferred embodiments the 3-dehydroquinate synthase protein is not a homolog, paralog or ortholog of Arabidosis thaliana 3-dehydroquinate synthase 2 (AtDHQS2), and, preferably, is not a protein consisting of an amino acid sequence with at least 100% sequence identity to an AtDHQS2 (SEQ ID NO: 5). In some more preferred embodiments, the degree of sequence identity to SEQ ID NO: 1 is higher than the degree of sequence identity to AtDHQS2 (SEQ ID NO 5).

In other preferred embodiments the 3-dehydroquinate synthase protein is not a homolog, paralog or ortholog of 3-dehydroquinate synthase 2 (DHQS2), and, preferably, is not a protein consisting of an amino acid sequence with at least 100% sequence identity to an DHQS2 of any one of SEQ ID Nos 5 to 7. In some more preferred embodiments, the degree of sequence identity to SEQ ID NO: 1 is higher than the degree of sequence identity to AtDHQS2 (SEQ ID NO 5).

In certain embodiments of the invention the 3-dehydroquinate synthase protein is a mature 3-dehydroquinate synthase protein not comprising an organelle transport signal, preferably not comprising a plastid transport signal.

In accordance with the aspects and embodiments of the invention the 7dSh, or the derivative thereof, is used by applying the 7dSh, or the derivative thereof, to the ground of the field, and preferably the 7dSh is not brought into direct contact with overground plant parts of the crop. For example, the or the 7dSh, or derivative thereof, is applied to the crop field as a crop protection formulation, such as a dispersion, powder or granule.

In preferred embodiments of the method of the invention step (a) comprises a step of screening at least two crop plant species or crop species variety of the desired crop for a detectable expression of the 3-dehydroquinate synthase protein, and the selecting of the crop plant species or crop species variety of the desired crop with a higher expression of the 3-dehydroquinate synthase protein.

In another preferred embodiment of the invention the detectable expression of the 3-dehydroquinate synthase protein is a detectable expression of the 3-dehydroquinate synthase protein in the roots of the plant.

In preferred embodiments of the method of the invention step step (a) comprises a step of increasing expression of the 3-dehydroquinate synthase in the crop plant. For example, the expression of the 3-dehydroquinate synthase is increased by introducing into the crop plant a genetic construct for the expression of the 3-dehydroquinate synthase in the crop plant. In certain embodiments the expression is a transgenic protein expression of the 3-dehydroquinate synthase only in the roots of the crop plant. In some embodiments of the invention, increasing expression of the 3-dehydroquinate synthase involves transgenic expression of a 3-dehydroquinate synthase protein comprising a organelle localization signal, such as a plastid localization signal. The transgenic expression may involve a genetic construct comprising a ubiquitous, tissue specific, and/or inducible promoter operably linked to an expressible sequence encoding the 3-dehydroquinate synthase protein.

In **a second aspect,** the invention pertains to a method for producing a herbicide resistant crop plant, the method comprising the steps of
- Introducing transgenic constructs for the expression of a 3-dehydroquinate synthase protein in a crop plant into multiple candidate crop plants or crop plant species;
- Detecting the expression of the 3-dehydroquinate synthase protein in the candidate crop plants;
- Selecting the crop plant with the highest expression of the 3-dehydroquinate synthase protein and/or with highest and most root-restricted expression of the 3-dehydroquinate synthase protein.

In **a third aspect,** the invention pertains to a plant cell nucleus, a plant cell, a plant tissue, propagation material, seed, pollen, progeny, or a plant part, resulting in a plant with increased herbicide tolerance or resistance after regeneration; or a plant with increased herbicide tolerance or resistance; or a part thereof; with said herbicide tolerance or resistance increased as compared to a corresponding wild type produced by a method as defined herein or being transformed with the nucleic acid molecule encoding for a 3-dehydroquinate synthase protein.

Preferably, the transgenic plant cell nucleus, transgenic plant cell, transgenic plant or part thereof, of the invention is derived from a monocotyledonous plant. In some embodiments the transgenic plant cell nucleus, transgenic plant cell, transgenic plant or part thereof of the invention is derived from a dicotyledonous plant.

It is preferred that in this aspect the corresponding plant is selected from the group consisting of corn (maize), wheat, rye, oat, triticale, rice, barley, soybean, peanut, cotton, oil seed rape, including canola and winter oil seed rape, manihot, pepper, sunflower, sugar cane, sugar beet, flax, borage, safflower, linseed, primrose, rapeseed, turnip rape, ta- getes, solanaceous plants comprising potato, tobacco, eggplant, tomato; Vicia species, pea, alfalfa, coffee, cacao, tea, Salix species, oil palm, coconut, perennial grass, forage crops and Arabidopsis thaliana. Such are the preferred crop plants in accordance with the invention in all aspects and embodiments. More preferably a crop plant in accordance with the invention is selected from the group consisting of corn, soy, oil seed rape (including canola and winter oil seed rape), cotton, wheat and rice.

In **a fourth aspect,** the invention pertains to a transgenic plant comprising one or more of plant cell nuclei or plant cells, progeny, seed or pollen or produced by a transgenic plant of the invention.

In **a fifth aspect,** the invention pertains to a method for growing the plant as defined in any one of claims 15 to 20, while controlling weeds in the vicinity of said plant, said method comprising the steps of:
- growing said plant on a field; and
- applying a herbicide composition comprising a 7dSh, or a derivative thereof, on the field, wherein the herbicide normally inhibits the nutrient uptake through the roots of a plant, at a level of the herbicide that would inhibit the growth of a corresponding wild-type plant.

Preferably, the herbicide composition is only applied to the roots of the plants on the field, for example wherein the herbicide composition is in crop protection formulation, such as a liquid or solid formulation, preferably in granule form.

In **a sixth aspect,** the invention pertains to a combination useful for weed control, comprising (a) a nucleic acid molecule for a transgenic expression of the 3-dehydroquinate synthase protein, which nucleic acid molecule is capable of being expressed in the roots of a crop plant to thereby provide to that crop plant a tolerance to a 7dSh, or a derivative thereof, containing herbicide composition; and (b) a herbicide composition comprising 7dSh, or a derivative thereof.

In **a seventh aspect,** the invention pertains to a use of a combination useful for weed control, comprising (a) a nucleic acid molecule for a transgenic expression of the 3-dehydroquinate synthase protein, which nucleic acid molecule is capable of being expressed in the roots of a crop plant to thereby provide to that crop plant tolerance to a 7dSh, or a derivative thereof, containing herbicide composition; and (b) a herbicide composition comprising 7dSh, or a derivative thereof, to control weeds at a plant cultivation site.

In some embodiments of the invention, the methods as described do not comprise a step of crossing and selecting whole genomes.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1**: shows the enzymatic activity of mature AtDHQS1 (mDHQS1) and the AtDHQS1 variant still containing the transit peptide (cTP) for plastid import (pDHQS1) are not inhibited by 7dSh. (A) Schematic representation of mDHSQ1 and pDHQS1 both containing a C-terminal (His)6 tag for purification after heterologous expression of the recombinant proteins in E.coli. aa: amino acid. (B) Relative activity of mDHQS1 (left) and pDHQS1 (right) in the presence of the indicated concentrations of 7dSh. The activity in the absence of 7dSh was set to 1. The enzymatic assay was carried out using 50 ng purified recombinant enzyme each.

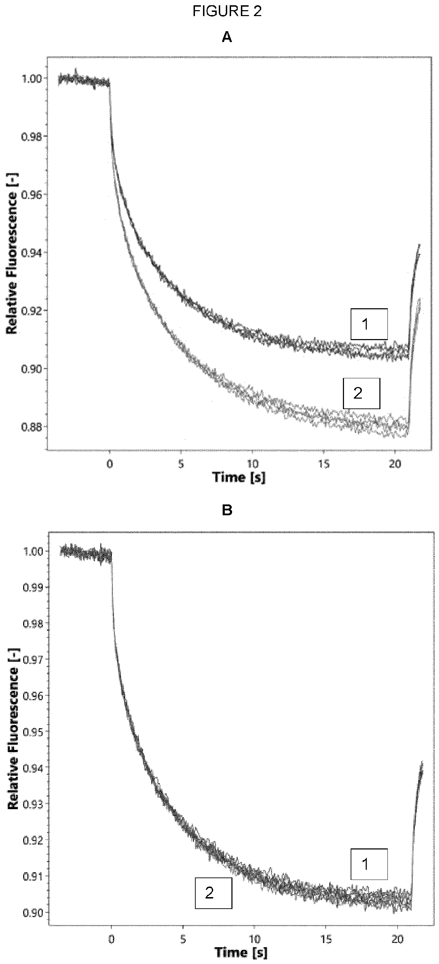

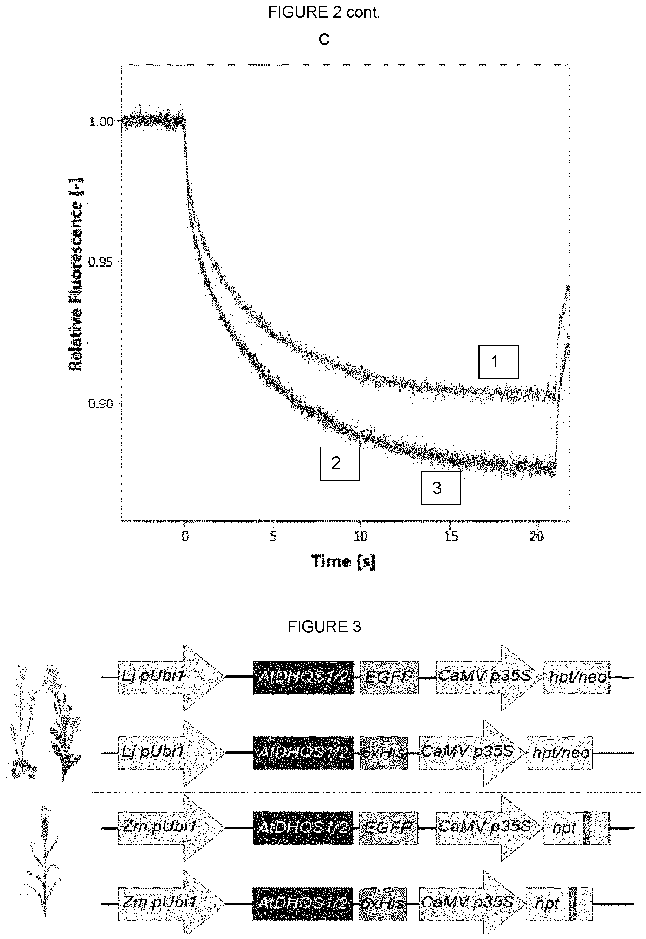

**Figure 2****:** shows DAHP, the natural substrate of mAtDHQS1, but not 7dSh, proposed to be a competitive inhibitor of DAHP, binds to mAtDHQS1 as determined by MicroScale Thermophoresis (MST). (A) MST fluorescence spectrum of RED dye-labelled mAtDHQS1 (50 nM) in the absence of DAHP (blue line, marked as line 1) or in the presence of 500 µM DAHP (green line, marked as line 2). The downward shift of the spectrum in the presence of DAHP demonstrates its binding to the enzyme. (B) MST fluorescence spectrum of RED dye-labelled mAtDHQS1 (50 nM) in the absence of 7dSh (blue line marked as line 1) or in the presence of 500 µM 7dSh (green line marked as line 2). No downward shift is detectable, showing that there is no binding. (C) MST fluorescence spectrum of RED dye-labelled mAtDHQS1 (50 nM) in the absence of 7dSh and DAHP (blue line, marked as line 1), in the presence of 250 µM DAHP (green line marked as line 2) or 250 µM DAHP and 250 µM 7dSh (red line marked as line 3). The DAHP binding-induced downward shift of the spectrum is not altered by 7dSh, indicating that there is no competition of 7dSh with DAHP for the substrate pocket in mAtDHQS1.
**Figure 3****:** shows Schematic representation of the AtDHQS1 and AtDHQS2 constructs (AtDHQS1/2) being used for the generation of transgenic Arabidopsis thaliana, rapeseed (Brassica napus) and barley (Hordeum vulgare) lines. Lj pUbi1: promoter of ubiquitin 1 gene from Lotus japonicus; EGFP: enhanced green fluorescence protein gene; 6xHis: (histidine)6 gene; CaMV p35S: promoter of the 35S genes from cauliflower mosaic virus; hpt: hygromycin phosphotransferase gene mediating hygromycin resistance; neo: neomycin resistance gene; Zm pUbi1: promoter of the ubiquitin 1 gene from Zea mays (corn).

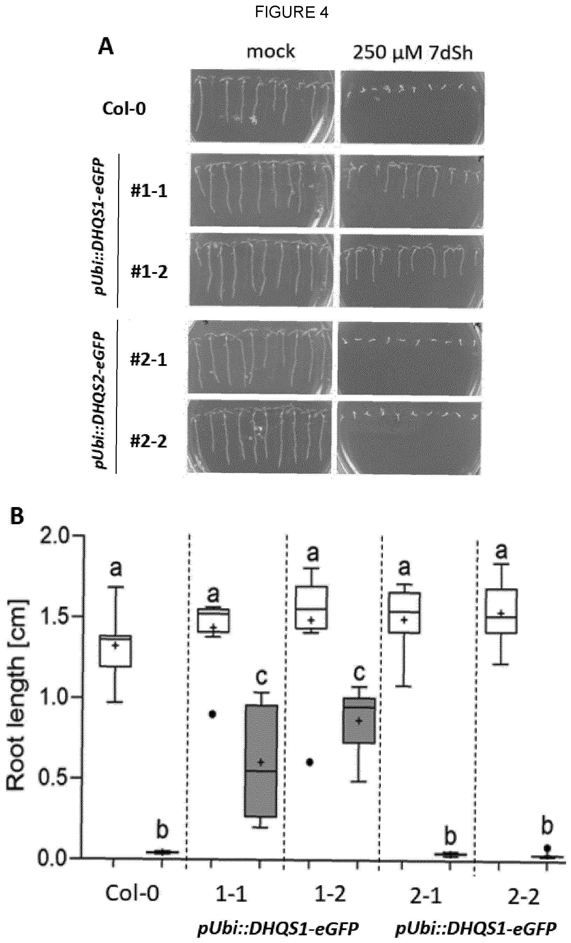

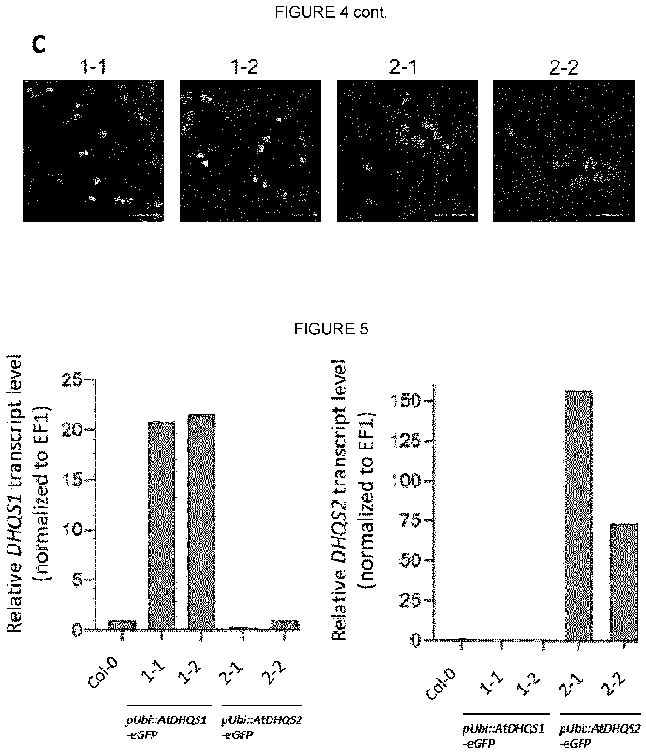

**Figure 4****:** shows Ectopic expression of AtDHQS1 but notAtDHQS2 leads to 7dSh-tolerant transgenic Arabidopsis plants. (A) Growth of 3-days-old wild type plants (Col-0) or two independent transgenic lines each ectopically overexpressing either EGFP-tagged AtDHQS1 (1-1, 1-2) or EGFP-tagged AtDHQS2 (2-1, 2-2) after 3 days on control plates (mock) or on plates containing 250 µM 7dSh. (B) As in (A) but the root length was quantified. n = 10 different, lower case letters mean statistically significant differences between the data sets (ANOVA test). (C) Subcellular localization of EGFP-tagged AtDHQS1 (lines 1-1 and 1-2) or EGFP-tagged AtDHQS2 (lines 2-1 and 2-2) in chloroplasts (red fluorescence) of 3-days-old transgenic Arabidopsis plants. Note that AtDHQS1-GFP shows a diffuse and AtDHQS2-GFP a dotted distribution within the chloroplast. Bar = 20 µm.
**Figure 5****:** shows Transcripts of AtDHQS1-GFP and AtDHQS2-GFP strongly accumulate in transgenic Arabidopsis lines described in Figure 4. (Left) relative AtDHQS1 transcript levels in the indicated transgenic Arabidopsis lines as determined by qRT-PCR using AtDHQS1 gene-specific primers. AtDHQS1 transcript levels are about ten times higher than in wild type (Col-0). (Right) relative AtDHQS2 transcript levels in the indicated transgenic Arabidopsis lines as determined by qRT-PCR using AtDHQS2 gene-specific primers. AtDHQS2 transcript levels are about hundred times higher than in wild type (Col-0).

The sequences show:
**SEQ ID NO: 1 (AtDHQS1)** 
MAANTISLSNVAASKNLNSFQSRAFIAPPTIFFPVASAKSKPGELSLSSTTLSRSRVRAGASQLM
NEPLNDQRSISSPTVVEVDLGDRSYPIYIGAGLLDHSELLQRHVHGKRVLVVTNDRVAPLYLDKT
IDALTRGNPNVTVESVILPDGEKYKDMDTLMKVFDKAIESRLDRRCTFVALGGGVIGDMCGYAA
ASYLRGVNFIQIPTTVMAQVDSSVGGKTGINHRLGKNLIGAFYQPQCVLVDTDTLNTLPDREMA
SGLAEVIKYGLIRDAEFFEWQEKNIEALLARDPAALAFAIKRSCENKADVVSQDEKESGLRATLN
LGHTFGHAIETGFGYGEWLHGEAVAAGTVMAVDMSYRLGWIDESIVKRVNKILVRAKLPTTPPE
SMTVSMFKSIMAVDKKVADGLLRLILLKGPLGNCVFTGDYDREALDATLRAFSKS
**SEQ ID NO: 2** Brassica napus DHQS1 ortholog; tr|A0A654FCZ2|A0A654FCZ2_ARATH 3-dehydroquinate synthase OS=Arabidopsis thaliana OX=3702 GN=At3g28760 PE=4 SV=1 
MALPLVSSVSYIKSSNQFHCSFSLRKEKFELSSFFIVLCSHFSYVDTAERLELDRLLLWSSHQKIR
FSKRTFSQRIVVKMSASTLPMNLGKAKKVWIWTMCKEVMTVAVERGWNTFIFSSDNRKLSNE
WSSIALMDTLFIEEKKVIDGTGNVVASVFEVSTPEELRSLNIENEQIENIVLDFLDWKSIPAENLVA
ALQGSEKTVFAVSNTPSEAKLFLEALEHGLGGIILKSEDVKAVLDLKEYFDKRNEESDTLSLTEAT
ITRVQMVGMGDRVCVDLCSLMRPGEGLLVGSFARGLFLVHSECLESNYIESRPFRVNAGPVHA
YVAVPGGKTCYLSELRTGREVIVVDQKGKQRTAVVGRVKIEKRPLIVVEAKLSTKEEETVYSIILQ
NAETVALVTPHQVNSSGRTAVPVTSLKPGDQVLIRLQGGARHTGIEIQEFIVEN
**SEQ ID NO: 3** Brassica rapa DHQS1 ortholog; tr|M4F7X4|M4F7X4_BRACM 3-dehydroquinate synthase domain-containing protein OS=Brassica campestris OX=3711 GN=BRARA_I00922 PE=3 SV=1 
MAANAVSLSISSTVASSKTLHSLQAQTRGLIPPPTISFPKSSSSLSSTTISISRSRVRAGPSQLVN
EPANAMATPPTIVEVDLGSRSYPIYIGAGLLDQSHLLQRHVHGKKVLVVTNERVAPLYLDKTVHA
LTIGNPNVTVESVILPDGEKYKDMDTLMKVFDKAIESRLDRRSTFVALGGGVIGDMCGYAAASY
LRGVNFIQIPTTVMAQVDSSVGGKTGINHRLGKNLIGAFYQPQCVLVDTDTLNTLPDREMASGL
AEVIKYGLIRDADFFEWQEKNIEALLARDPAALAYAIKRSCENKADVVSQDEKESGLRATLNLGH
TFGHAIETGFGYGEWLHGEAVAAGTVMAVDMSHRLGWIDDSIVERVNNILKRAKLPTTPPESMT
VSMFKSIMAVDKKVADGLLRLILLKGPLGNCVFTGDYDREALDATLRAFSKS
**SEQ ID NO: 4** Hordeum vulgare DHQS1 ortholog; UPI000B478687 status=inactive 
LIGGNRTQPPPPPPPPFRDQTTPMAASASSLLAAAASSSSRAAISVRRPRAAPAAAAASVPSSSR
SSFTPLRASPARALRSRVVASAAPAMQPPAVSRVSTVVDVLGDRSYPIYIGPGLLNEPDLLQR
HVIGKRVLVVTNTTVAPLYLEKVTWALTHQNPNVSVESVILPDGEKYKDMDTLMKVFDKAVESR
LDRRCTFVALGGGVIGDMCGFAASAFLRGVNFIQIPTTLMAQVDSSVGGKTGINHPLGKNLIGA
FYQPQCVLIDTETLNTLPDRELASGVAEVVKYGLIRDAPFFEWQEKNMAAILAREPSALTYAIKR
SCENKAEVVAQDEKESGLRATLNLGHTFGHAIETGLGYGEWLHGEAVAAGTVMAADMSYRLG
WIDESIKKRTFDILDQAKLPVTPPKGMTVEKFRNIMAVDKKVADGLLRLILLKGPLGGCVFTGEY
DRKALDETLRAFCDE
**SEQ ID NO:5 (AtDHQS2)** 
MALPLVSSVSYIKSSNQFHCSFSLRKEKFELSSFFIVLCSHFSYVDTAERLELDRLLLWSSHQKI
RFSKRTFSQRIVVKMSASTLPMNLGKAKKVWIWTMCKEVMTVAVERGWNTFIFSSDNRKLSNE
WSSIALMDTLFIEEKKVIDGTGNVVASVFEVSTPEELRSLNIENEQIENIVLDFLDWKSIPAENLVA
ALQGSEKTVFAVSNTPSEAKLFLEALEHGLGGIILKSEDVKAVLDLKEYFDKRNEESDTLSLTEA
TITRVQMVGMGDRVCVDLCSLMRPGEGLLVGSFARGLFLVHSECLESNYIESRPFRVNAGPVH
AYVAVPGGKTCYLSELRTGREVIVVDQKGKQRTAVVGRVKIEKRPLIVVEAKLSTKEEETVYSIIL
QNAETVALVTPHQVNSSGRTAVPVTSLKPGDQVLIRLQGGARHTGIEIQEFIVEN
**SEQ ID NO:** 6 **Brassica rapa** DHQS2 ortholog; >tr|A0A397Z2M7|A0A397Z2M7_BRACM 3-dehydroquinate synthase OS=Brassica campestris OX=3711 GN=BRAA06T26953Z PE=4 SV=1 
MALPLVSSVSYIQSSSTQLHCFPLRKERFGLYRLQTWSSHHSSTRLIRKKTFPQRLAVRMSASS
TLPMNLQRSKKVWIWTECKEVMTTAVERGWNTFVFSSDSLQLSDDWSSVALVDALFIDERQVT
DGTGRVVAAVFEVSTPEELQMLKIENEQAENIVLGLLDWKSIPAENLVAALQGSEKTVFAISSTPS
EAKLYLEALEHGLGGIILKTEDVKAVLDLKEYFDKRGEESDTLSLTEATITRIEMVGMGDRVCVDL
CSLMRPGEGLLVGSFARGLFLVHSECLESNYIASRPFRVNAGPVHAYVAVPGGRTCYLSELRTG
RQVIVVDQKGKQRTAVVGRVKIEKRPLILVEAKLSGEEDETSFSTILQNAETVALVTPHQVNSSG
KTAVPVTSLKLGDQVLIRLQGGARHTGIEIQEFIVEN
**SEQ ID NO: 7** Hordeum vulgare DHQS2 ortholog; >tr|A0A287PPD1|Release 2021_04/2021_04129-Sep-2021

KTGANDQMPHPEIPQRNSMAVSCSSSSSSILGSAKSLACFHTSNLAPQRTASARSRLQSIKMR
SAPAAPGESKKTVWVWTENRQVMTAAVERGWNTFLFGSKDVGKDWSSTARIHPLFIDGLEIFD
EGNQKVATISEISSPSELQLIQPDNVEVQNTVIHFRGDWQVIPAENIVAAFQGCAGTVLAVSTNS

TEAQVFLEALEQGLNGVVLKVEDMDDIIKLKDYFDRRNEAKSQLQLTKATVSKVEVVGMGDRV
CVDLCSIMRPGEGLLVGSYARGMFLVHSECLETNYIASRPFRVNAGPVHAYVTVPGDKTSYLSE
LQSGREVIVVDQNGLWRTSIVGRVKIESRPLILVEAKENSGNGTYSIFLQNAETVALIGPDRGSG
GRTAIPVTSLKVGDEVLVRKQGGARHTGIEIQEFIVE

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: AtDHQS1 is not the target of 7dSh

In the published work (Rath et al., 2022), in addition to the recombinant and purified DHQS from Anabaena variabilis, the recombinant AtDHQS1 from Arabidopsis thaliana was used in a version that still has the 61 amino acid long transit peptide for plastid import (pAtDHQS1). The mature i.e. biologically relevant enzyme (mAtDHQS1) with 381 amino acids is thus significantly shorter than the previously used version with 442 amino acids (Figure 1 A). To rule out any possible discrepancies in the activities, mAtDHQS1 was also produced recombinantly in E. coli, purified and examined for its enzymatic properties in comparison to pAtDHQS1. Both enzyme versions convert their substrate DAHP with a similar Vmax (mAtDHQS1: 3.4 nmol DAHP min-1 and µg protein-1, pAtDHQS1: 1.5 nmol DAHP min-1 and µg protein-1) and KM (mAtDHQS1: 8.3 µM, pAtDHQS1: 2.9 µM). These values agree well with the data published by Rath et al. (2022).

Now the effect of 7dSh on the activity of mAtDHQS1 and pAtDHQS1 was tested in a concentration range previously reported to be effective (100 to 1000 µM; Rath et al., 2022). However, as shown in Figure 1B, there is no effect of 7dSh on both enzyme versions at any concentration and protein amount tested. This suggest that 7dSh is not acting on the enzymatic activity of AtDQSH1, whichever enzyme variant was investigated. This surprising result prompted the inventors to use the very sensitive MicroScale Thermophoresis (MST) technology to check whether 7dSh - and as a positive control the substrate DAHP - actually binds to mAtDHQS1 physically. While DAHP has a very high affinity to mAtDHQS1 (KD: 3.3 × 10-5 M; Fig. 2 A for the spectrum), there is no interaction of 7dSh with AtDHQS1 (Fig. 2 B). Furthermore, MST competition assays showed that 7dSh is not able to displace DAHP from its binding to mAtDHQS1 (Fig. 2 C). The results, which are consistent in their significance using two fundamentally different methods (enzyme assay, MST) only allow the conclusion that 7dSh, as a postulated competitive inhibitor (Rath et al., 2022), cannot have mAtDHQS1 as its target enzyme. In our view, the effect of 7dSh on plant metabolism, including the shikimate pathway, must have a novel biochemical and molecular-mechanistic background.

### Example 2: AtDHQS1-overexpressing plants show partial tolerance to 7dSh

To further investigate the function of AtDHQS1, transgenic Arabidopsis plants, which overexpress AtDHQS1 under the control of the ubiquitin 1 promoter of Lotus japonicus (Lj pUbi1) were generated. In contrast to the 35S promoter, the Lj pUb1 promoter has a much lower risk of silencing and mediates an intermediate to high, constitutive expression of the transgene in all plant tissues. For the generation of transgenic lines overexpressing AtDHQS1 in barley the ubiquitin 1 promoter from maize (Zm pUb1) was used. The AtDHQS1 DNA constructs were designed in a way that they contain a selection marker (hygromycin for transgenic Arabidopsis and barley; kanamycin for rapeseed/canola) and that the accumulating AtDHQS1 enzyme carries either a C-terminal GFP-tag or (His)₆-tag for immunodetection and in planta subcellular localization analysis (Fig. 3). The same set of constructs was generated forAtDHQS2, which has a 30 % similarity to AtDHQS1 at amino acid sequence level (Fig. 3). After transformation of the constructs into wild type Arabidopsis thaliana and very elaborate selection and segregation analyses, several independent homozygous transgenic lines were obtained expressing the GFP fusion of AtDHQS1 and AtDHQS2. qRT-PCR analyses using AtDHQS1- or AtDHQS2-specific primers revealed that AtDHQS1-GFP transcripts accumulated 5 to 10 times and AtDHQS2-GFP transcripts still a power of ten higher above wild type level (Fig. 5). Both GFP fusion proteins were found to be located inside the chloroplast as predicted but with a distinct subplastidary localization pattern (Fig. 4 C).

Next, comparative root growth experiments were performed on growth medium without (mock) or with 250 µM 7dSh using 3-days-old individuals of Arabidopsis wild type seedlings or individuals of two independent transgenic lines, which overexpress either AtDHQS1-GFP or AtDHQS2-GFP. Whereas root growth of wild type seedlings was completely inhibited by 7dSh, the seedlings of transgenic lines overexpressing AtDHQS1-GFP showed a partial but not complete tolerance to the active substance (Fig. 4 A/B). In contrast and although AtDHQS2-GFP transcripts accumulated to a 10 times higher amount than AtDHQS1 transcripts (Fig. 5), there was no tolerance to 7dSh in the seedlings of AtDHQS2-GFP transgenic lines (Fig. 4 A/B). The latter observation indicates that AtDHQS2 has no role in mediating 7dSh effects in Arabidopsis. It is important to note that the overexpression of AtDHQS1-GFP (and AtDHQS2-GFP) had no effect on root growth under normal conditions (mock in Fig. 4 A/B), indicating that there is no apparent trade-off with respect to seedling growth and development, when these enzymes over-accumulate in the plant.

The finding that AtDHQS1 is not the target of 7dSh, but that the overexpression of AtDHQS1 (-GFP) causes partial tolerance to 7dSh in Arabidopsis seems contradictory, and is in stark contrast to the findings of the prior art. Without being bound to theory, overexpression of AtDHQS1 could lead to a metabolic imbalance in the plant, and thereby could indirectly influence the amount, constitution, intracellular localization, biochemical and physiological activity of counteracting enzymes or proteins, one of which potentially being the true target for 7dSh. This hypothesis is in agreement with a recent finding that a strong accumulation of sedoheptulose was detectable in the extracts after exposure of Arabidopsis thaliana, Brassica napus and Hordeum vulgare seedlings to 7dSh. The accumulation was up to 60 times higher than in the untreated seedlings of the three species. Sedoheptulose is a ketoheptose - a monosaccharide with seven carbon atoms and a ketone functional group and one of the few heptoses found in nature. Sedoheptulose is not a metabolite of the shikimate pathway and can in no way serve as substrate for AtDHQS1.

All these results indicate that the effect of 7dSh on plant development is based on a different molecular-biochemical mechanism than originally assumed.

## Claims

1. **A method for controlling unwanted vegetation on a crop field used for growing** a **crop plant,** the method comprising the steps of:
(a) Selecting a crop plant from a crop plant species or from a crop plant species variety of a desired crop having a detectable expression of a 3-dehydroquinate synthase (DHQS) protein;
(b) Growing the selected crop plant on the field;
(c) Controlling unwanted vegetation on the field by using 7-deoxy-sedoheptulose (7dSh), or a derivative thereof, on the field.

2. The method of claim 1, wherein the DHQS protein is a homolog, paralog or ortholog of *Arabidosis thaliana* DHQS 1 (AtDHQS1), or is a protein consisting of an amino acid sequence with at least 80% sequence identity to an AtDHQS1 (SEQ ID NO: 1), or is a homolog, paralog or ortholog of DHQS 1 (DHQS1) of another plant species, or is a protein consisting of an amino acid sequence with at least 80% sequence identity to an DHQS1 selected from any one of SEQ ID NO: 2 to 4.

3. The method of claim 1 or 2, wherein the DHQS protein is not a homolog, paralog or ortholog of *Arabidosis thaliana* DHQS 2 (AtDHQS2), and, preferably, is not a protein consisting of an amino acid sequence with at least 100% sequence identity to an AtDHQS2 (SEQ ID NO: 2).

4. The method of any one of claims 1 to 3, wherein step (a) comprises a step of screening at least two crop plant species or crop species variety of the desired crop for a detectable expression of the DHQS protein, and the selecting of the crop plant species or crop species variety of the desired crop with a higher expression of the DHQS protein.

5. The method of any one of claims 1 to 4, wherein the detectable expression of the DHQS protein is a detectable expression of the DHQS protein in the roots of the plant.

6. The method of any one of claims 1 to 5, wherein step (a) comprises a step of increasing expression of the DHQS in the crop plant.

7. The method of claim 6, wherein the expression of the DHQS is increased by introducing into the crop plant a genetic construct for the expression of the DHQS in the crop plant.

8. The method of any one of claims 6 or 7, wherein the transgenic expression involves a genetic construct comprising a ubiquitous, tissue specific, and/or inducible promoter operably linked to an expressible sequence encoding the DHQS protein.

9. **A method for producing a herbicide resistant crop plant,** the method comprising the steps of
(a) Introducing transgenic constructs for the expression of a DHQS protein in a crop plant into multiple candidate crop plants or crop plant species;
(b) Detecting the expression of the DHQS protein in the candidate crop plants;
(c) Selecting the crop plant with the highest expression of the DHQS protein and/or with highest and most root-restricted expression of the DHQS protein.

10. **A plant cell nucleus, a plant cell, a plant tissue, propagation material, seed, pollen, progeny, or a plant part,** resulting in a plant with increased herbicide tolerance or resistance after regeneration; or a plant with increased herbicide tolerance or resistance; or a part thereof; with said herbicide tolerance or resistance increased as compared to a corresponding wild type produced by a method as defined in claim 9 or being transformed with the nucleic acid molecule encoding for a DHQS protein as recited in any one of the preceding claims.

11. The transgenic plant cell nucleus, transgenic plant cell, transgenic plant or part thereof of claim 10, wherein the plant is a crop plant and preferably is selected from the group consisting of corn, soy, oil seed rape (including canola and winter oil seed rape), cotton, wheat and rice.

12. **A transgenic plant** comprising one or more of plant cell nuclei or plant cells, progeny, seed or pollen or produced by a transgenic plant of claim 10 or 11.

13. **A method for growing the plant as defined in any one of claims 10 to 12,** while controlling weeds in the vicinity of said plant, said method comprising the steps of:
(a) growing said plant on a field; and
(b) applying a herbicide composition comprising a 7dSh, or a derivative thereof, on the field, wherein the herbicide normally inhibits the nutrient uptake through the roots of a plant, at a level of the herbicide that would inhibit the growth of a corresponding wild-type plant.

14. **A combination useful for weed control,** comprising (a) a nucleic acid molecule for a transgenic expression of the DHQS protein as recited in any one of claims 1 to 8, which nucleic acid molecule is capable of being expressed at least in the roots of a crop plant to thereby provide to that crop plant tolerance to a 7dSh, or a derivative thereof, containing herbicide composition; and (b) a herbicide composition comprising 7dSh, or a derivative thereof.

15. **Use of a combination useful for weed control,** comprising (a) a nucleic acid molecule for a transgenic expression of the DHQS protein as recited in any one of claims 1 to 8, which nucleic acid molecule is capable of being expressed in the roots of a crop plant to thereby provide to that crop plant tolerance to a 7dSh, or a derivative thereof, containing herbicide composition; and (b) a herbicide composition comprising 7dSh, or a derivative thereof, to control weeds at a plant cultivation site.
